# EUROPÄISCHE PATENTANMELDUNG

(11) **EP 1 223 539 A2**
(43) Veröffentlichungstag der Anmeldung: **17.07.2002**
(21) Anmeldenummer: 01129451.9
(22) Anmeldetag: 10.12.2001
(51) Int. Cl.: G06K 9/00, G07C 9/00

(54) **Authentisierung einer Person mittels Handerkennung**

(30) Priorität: 09.01.2001 DE 10100616
(71) Anmelder: SIEMENS AKTIENGESELLSCHAFT, 80333 München (DE)
(72) Erfinder: Heger, Hans Jörg, 80807 München (DE); Küpper, Wolfgang, Dr., 80796 München (DE)

(57) **Zusammenfassung**

Es wird ein Antrieb (1) für einen beweglich an einem ortsfesten Bauteil gelagerten Flügel beschrieben. Der Antrieb (1) weist ein Antriebsgehäuse (2) auf, in welchem ein elektrischer Antriebsmotor (3), eine elektronischen Steuerung (7), ein Abtriebsglied (5), welches aus einer Öffnung des Antriebsgehäuses (2) austritt und direkt oder indirekt mit dem beweglichen Flügel kraftschlüssig verbunden ist, sowie ggf. ein zwischen dem Antriebsmotor (3) und dem Abtriebsglied (5) geschaltetes Getriebe (4) angeordnet sind. Der Antrieb (1) weist eine elektrische Verbindung zu einem Bussystem auf.

Der Antrieb (1) weist eine Schnittstelle (8) zum Anschluss an ein Bussystem auf, wobei die Schnittstelle (8) im Antriebsgehäuse (2) integriert angeordnet ist.

## Beschreibung

Die Erfindung betrifft ein Verfahren, eine Anordnung und ein Programmprodukt zur Authentisierung einer Person mittels Handerkennung.

Zur Authentisierung von Personen können biometrische Verfahren angewendet werden, das heißt, der Benutzer wird anhand von Körpermerkmalen oder charakteristischen Verhaltensweisen erkannt. Ein etabliertes biometrisches Verfahren ist die Authentisierung durch die Erkennung der Form der Hand oder eines Teils der Hand. Die Handkonturerkennung wird bislang durchgeführt, indem die Hand auf eine Platte aufgelegt wird. Die Positionierung der Hand wird dabei durch physikalische Hilfsmittel, wie zum Beispiel kleine Stahlbolzen oder Ähnliches, vorgegeben. Anschließend wird durch Abtasten ein Bild der Hand aufgenommen und weiterverarbeitet. Auf der Grundlage dieses Bildes mit der definierten Handposition wird die Authentisierung der Person durchgeführt. Die beschriebenen Anordnungen zur Handerkennung nach dem Stand der Technik sind durch die Führung der Hand in eine definierte Erkennungsposition beschränkt.

Aus US 4 720 869 ist weiterhin eine Anordnung zur Handerkennung bekannt, bei der die Hand gleichzeitig aus zwei verschiedenen Richtungen abgetastet wird.

Schließlich sind aus US 5 533 177, US 5 751 843,
US 5 828 779, EP 0 560 779 B1, EP 0 713 592 B1,
EP 0 800 145 A2 und WO 98/38533 Anordnungen bekannt, bei denen Bewegungen von Hand und Arm abtastbar sind.

Wie jedes Authentisierungsverfahren sind auch die biometrischen Systeme Täuschungsversuchen ausgesetzt. Eine naheliegende Täuschung besteht darin, das biometrische System durch Modellnachbildungen zu täuschen. Im Falle der Handerkennung sind dreidimensionale Handimitate denkbar.

Davon ausgehend liegt der Erfindung die Aufgabe zugrunde, die Erkennungssicherheit bei der Handerkennung zu erhöhen, ohne dass der Erkennungsvorgang von der zu authentisierenden Person als unangenehm empfunden wird.

Diese Aufgabe wird durch eine Anordnung, ein Verfahren und ein Programmprodukt mit den Merkmalen der unabhängigen Ansprüche gelöst.

Dadurch, dass nicht nur ein Standbild der Hand aufgenommen wird, sondern auch der Übergang der Hand in eine zweite Position abgetastet wird, lässt sich eine Lebenderkennung realisieren. Das Abtasten kann dabei von einem Sensor vorgenommen werden, zum Beispiel von einer Videokamera oder von einer Standbildkamera, die eine hinreichend schnelle Bildfolge erzeugen kann.

Eine besonders sichere Lebenderkennung ergibt sich, wenn die Person nur authentisiert wird, wenn erkannt wird, dass die Hand beim Übergang von der ersten Position in die zweite Position in sich bewegt wird. Eine solche Bewegung in sich kann eine Bewegung der Finger oder des Handtellers sein, insbesondere eine Krümmungsbewegung. So kann vermieden werden, dass eine unzulässige Authentisierung dadurch erzielt wird, dass ein dreidimensionales Handimitat einfach hin und her bewegt wird.

Weiterhin kann bei der Handerkennung die für den Übergang benötigte Zeit berücksichtigt werden. Abhängig von unterschiedlichen Anfangs- und Endpositionen werden dazu unterschiedliche Übergangszeiten zugelassen, so dass eine Authentisierung nur bei einem Übergang stattfindet, der mit einer natürlichen Bewegungsgeschwindigkeit vollzogen wird.

Wenn man der zu authentisierenden Person die Art und Weise freistellt, auf die sie den Übergang zwischen der ersten und der zweiten Position vornimmt, kann durch die Erfassung des gewählten Übergangs in der Lernphase der Handerkennungsanordnung ein zusätzlicher Aspekt berücksichtigt und die Erkennungssicherheit durch eine Gestikerkennung noch weiter gesteigert werden. Die Person wird dann nur dann authentisiert, wenn erkannt wird, dass dieser bestimmte Übergang vorgenommen wird. Die Person wird sozusagen anhand einer eigentümlichen, individuellen Geste authentisiert.

Schließlich lässt sich die Hand auch noch in der zweiten Position abtasten und die Handerkennung auf Basis der für die zweite Position gewonnenen Abtastdaten durchführen. Vorzugsweise hat die Hand in der zweiten Position eine andere Handform einzunehmen als in der ersten Position. So kann etwa statt einer flachen Hand eine Faust vorgegeben werden. Durch diese Vorgehensweise können weitere charakteristische Merkmale abgetastet und bei der Handerkennung berücksichtigt werden, so dass die Erkennungssicherheit weiter erhöht wird.

Eine Anordnung, die eingerichtet ist, das Verfahren auszuführen, lässt sich zum Beispiel durch entsprechendes Programmieren und Einrichten einer Datenverarbeitungsanlage realisieren, der die Abtastdaten zuführbar sind.

Ein Programmprodukt für eine Datenverarbeitungsanlage, das Softwarecodeabschnitte enthält, mit denen eines der geschilderten Verfahren auf einer Datenverarbeitungsanlage ausgeführt werden kann, lässt sich durch geeignete Implementierung des Verfahrens in einer Programmiersprache ausführen. Die Softwarecodeabschnitte werden dazu gespeichert. Dabei wird unter einem Programmprodukt das Programm als handelbares Produkt verstanden. Es kann in beliebiger Form vorliegen, so zum Beispiel auf Papier, einem computerlesbaren Datenträger oder über ein Netz verteilt.

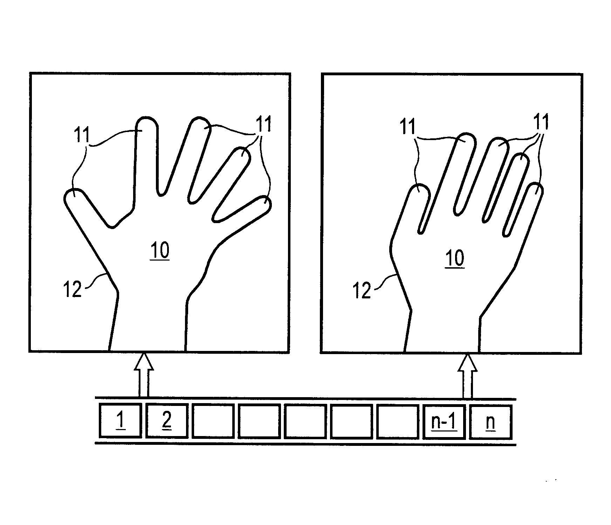

Weitere wesentliche und vorteilhafte Merkmale der Erfindung ergeben sich aus der Beschreibung eines Ausführungsbeispiels anhand der Zeichnung. Dabei zeigt
Figur 1 zwei Bilder einer Hand in zwei unterschiedlichen Positionen.

In einer bevorzugten Anordnung zur Realisierung der Erfindung wird von einer Videokamera die Hand 10 einer Person durch Aufnehmen eines Bildes abgetastet. Die Person wird aufgefordert, mit ihrer Hand 10 nacheinander eine erste und eine zweite Position einzunehmen. Die Videokamera erzeugt Bilder 1 bis n der Hand 10, wobei das Bild 1 in der ersten Position und das Bild n in der zweiten Position aufgenommen wird. Das Bild 1 der ersten Position und das Bild n der zweiten Position sind in Figur 1 vergrößert dargestellt. Mit den in schematisch dargestellten Bildern 2 bis n-1 wird der Übergang der Hand 10 von der ersten Position in die zweite Position abgetastet.

In Position 1 wird die Hand 10 als flache Hand 10 mit gespreizten Fingern 11 gehalten. Aufgrund der in dieser Position erhaltenen Abtastdaten wird eine Handerkennung durchgeführt, wobei aus der Kontur 12 der Hand 10 über einen Segmentierungsvorgang charakteristische Merkmale extrahiert werden. Diese Merkmale werden mit in einer Lernphase aufgenommenen Merkmalen für die Hände unterschiedlicher zu authentisierender Personen verglichen und es wird bei hinreichender Übereinstimmung eine Person vorläufig authentisiert.

Statt, wie beschrieben, zur Identifizierung kann das Verfahren auch zur Verifikation der Person eingesetzt werden. Dabei ist die behauptete Identität der Person bekannt und wird durch die Handerkennung verifiziert oder nicht verifiziert.

In einem weiteren Schritt wird festgestellt, ob die Hand 10 sich beim Übergang von der ersten in die zweite Position, vorzugsweise in sich, bewegt hat. Im dargestellten Beispiel erfolgt die Bewegung durch ein Zusammenlegen der in der ersten Position gespreizten Finger 11 der Hand 10. Ein solches Zusammenlegen wäre bei einem einfachen starren Handimitat nicht möglich. Dementsprechend erkennt die Handerkennung, dass es sich um die Hand 10 einer lebenden Person handelt.

Vorzugsweise wird auch die in der zweiten Position abgetastete Kontur 12 der Hand 10 einem Handerkennungsverfahren unterworfen.

Ist die Handerkennung insgesamt positiv verlaufen, so wird die Person authentisiert und es können Schritte eingeleitet werden, die abhängig von dem Einsatzort und -zweck der Handerkennung sind.

Allen Ausführungsformen der Erfindung ist der Vorteil zu Eigen, dass eine Lebenderkennung der Hand vorgenommen und damit die Erkennungssicherheit erhöht werden kann.

## Patentansprüche

1. Verfahren zur Authentisierung einer Person mittels Handerkennung, bei dem
- eine Hand (10) in einer ersten Position abgetastet wird,
- die Hand (10) in eine zweite Position übergeht,
- der Übergang zwischen den beiden Positionen abgetastet wird und
- eine Handerkennung durchgeführt wird.

2. Verfahren nach Anspruch 1,
bei dem eine Lebenderkennung auf Basis der für den Übergang gewonnenen Abtastdaten durchgeführt wird.

3. Verfahren nach Anspruch 1 oder 2,
bei dem die Handerkennung auf Basis der für die erste Position und der für den Übergang gewonnenen Abtastdaten durchgeführt wird.

4. Verfahren nach zumindest einem der vorhergehenden Ansprüche,
bei dem die Person nur authentisiert wird, wenn erkannt wird, dass die Hand (10) beim Übergang in sich bewegt wird.

5. Verfahren nach zumindest einem der vorhergehenden Ansprüche,
bei dem bei der Handerkennung die für den Übergang benötigte Zeit berücksichtigt wird.

6. Verfahren nach zumindest einem der vorhergehenden Ansprüche,
bei dem die Person nur authentisiert wird, wenn ein bestimmter Übergang erkannt wird.

7. Verfahren nach zumindest einem der vorhergehenden Ansprüche,
bei dem die Hand (10) in der zweiten Position abgetastet wird und die Handerkennung auch auf Basis der für die zweite Position gewonnenen Abtastdaten durchgeführt wird.

8. Verfahren nach zumindest einem der vorhergehenden Ansprüche,
bei dem die Hand (10) in der zweiten Position eine andere Handform einzunehmen hat als in der ersten Position.

9. Anordnung, die eingerichtet ist, ein Verfahren nach zumindest einem der Ansprüche 1 bis 8 auszuführen.

10. Programmprodukt für eine Datenverarbeitungsanlage, das Softwarecodeabschnitte enthält, mit denen ein Verfahren nach zumindest einem der Ansprüche 1 bis 8 auf einer Datenverarbeitungsanlage ausgeführt werden kann.
